# FASCICULE DE BREVET EUROPEEN

(11) **EP 3 003 415 B1**
(45) Date de publication et mention de la délivrance du brevet: **16.05.2018**
(21) Numéro de dépôt: 14731811.7
(22) Date de dépôt: 03.06.2014
(51) Int. Cl.: A61L 27/10, A61L 27/22, A61L 27/24, A61L 27/54, A61L 27/16, A61L 27/20, A61L 27/30, A61L 27/18, A61L 27/44, A61L 27/56, A61L 27/58

(54) **IMPLANT A POROSITE CONTROLEE COMPRENANT UNE MATRICE REVETUE D'UN VERRE BIOACTIF OU D'UN MATERIAU HYBRIDE**
IMPLANTAT MIT KONTROLLIERTER POROSITÄT MIT EINER MATRIX MIT ABDECKUNG AUS BIOAKTIVEM GLAS ODER HYBRIDMATERIAL
IMPLANT WITH CONTROLLED POROSITY COMPRISING A MATRIX COVERED BY A BIOACTIVE GLASS OR BY A HYBRID MATERIAL

(30) Priorité: 03.06.2013 FR 1355057
(43) Date de publication de la demande: 13.04.2016
(73) Titulaire: Université Clermont Auvergne, 63000 Clermont-Ferrand (FR); Centre National de la Recherche Scientifique, 75794 Paris Cedex 16 (FR)
(72) Inventeur: LAO, Jonathan, Claude, Alexandre, 63960 Veyre-Monton (FR); LACROIX, Joséphine, 72000 Le Mans (FR); JALLOT, Edouard, Daniel, Albert, 63360 Saint-beauzire (FR); DIEUDONNE, Xavier, 63122 Ceyrat (FR)
(74) Mandataire: Gevers & Orès
(86) Numéro de dépôt international: PCT/IB2014/061915
(87) Numéro de publication internationale: WO 2014/195864

(56) Documents cités:
- HONG LI ET AL: "Enhancement of the osseointegration of a polyethylene terephthalate artificial ligament graft in a bone tunnel using 58S bioglass", INTERNATIONAL ORTHOPAEDICS, SPRINGER, BERLIN, DE, vol. 36, no. 1, 17 mai 2011 (2011-05-17), pages 191-197, XP019998043, ISSN: 1432-5195, DOI: 10.1007/S00264-011-1275-X
- ANKE LISA METZE ET AL: "Gelatin Coated 45S5 Bioglass -Derived Scaffolds for Bone Tissue Engineering", BONE AND BIOMATERIALS FOR BONE TISSUE ENGINEERING : SPECIAL TOPIC VOLUME WITH INVITED PEER REVIEWED PAPERS; [KEY ENGINEERING MATERIALS; VOL. 541], TTP, TRANS TECH PUBL, CH, vol. 541, 1 January 2013 (2013-01-01), pages 31-39, XP008170988, DOI: 10.4028/WWW.SCIENTIFIC.NET/KEM.541.31 ISBN: 978-3-03785-582-9 [retrieved on 2013-02-01]

## Description

L'invention concerne un matériau d'implant pour le comblement de défauts osseux, la régénération osseuse et l'ingénierie tissulaire de l'os, un implant comprenant ce matériau, un procédé de fabrication d'un tel implant.

Le vieillissement global de la population et les troubles du système ostéo-articulaire qui l'accompagnent rendent nécessaire le développement de matériaux de substitution des tissus osseux de haute performance. 18 milliards d'euros de frais de santé sont en effet dépensés chaque année en France pour les maladies du système ostéo-articulaire et dentaires, les troubles musculo-squelettiques sont les pathologies professionnelles les plus répandues dans les pays industrialisés, tandis que l'ostéoporose se développe chez les patients âgés ; ces faits dessinent les contours d'un enjeu sociétal et économique majeur et expliquent la demande croissante en biomatériaux, implants à durée de vie accrue capables de combler les pertes osseuses.

Le recours aux greffes étant limité, et les matériaux d'origine animale pouvant poser des problèmes de biocompatibilité ou des risques d'infection, les efforts de recherche visent à élaborer des biomatériaux synthétiques capables de promouvoir la régénération osseuse.

On parle dans ce cas d'implants bioactifs : le matériau implanté n'est pas simplement destiné à combler de manière passive une perte osseuse en restant le plus inerte possible, mais au contraire il doit stimuler et participer activement au mécanisme de régénération osseuse. Ceci est particulièrement important dans le cas de larges défauts osseux, pour lesquels le mécanisme d'autoréparation ne fonctionne plus.

Actuellement les principaux matériaux bioactifs utilisés comme substituts osseux sont les « céramiques » bioactives, telles que les phosphates de calcium, et les verres bioactifs, également appelés « bioverres ».

Les premières céramiques bioactives ont été développées par L.L. Hench (L.L. Hench et al., J. Biomed. Mater. Res. 1971, 2, 117-141 ; L.L. Hench et al., J. Biomed. Mater. Res. 1973, 7, 25-42).

Les premiers verres bioactifs ont été préparés à partir de SiO₂, de P₂O₅, de CaO et de Na₂O. Les oxydes de silicium et de phosphore sont des formateurs de réseau qui participent à la cohésion du réseau vitreux. Les alcalins et alcalino terreux comme le sodium et le calcium ne présentent pas cette capacité et viennent modifier le réseau vitreux en y introduisant des ruptures de chaines qui sont à l'origine de la faible température de fusion de ces verres associée à un désordre structural accru. Leur présence a pour conséquence une plus grande réactivité des verres bioactifs à travers notamment leur corrosion dans un environnement aqueux. Cette réactivité permet la formation d'hydroxyapatite en milieu physiologique et favorise donc la reconstruction osseuse.

Le bioverre qui a été le plus étudié est un verre sodo-silico-phosphocalcique dit Bioglass® ou Bioverre de Hench. Sa composition de base est 45% SiO₂ - 24,5% CaO - 24,5% Na₂O - 6% P₂O₅, en masse par rapport à la masse totale de la composition. Les propriétés bioactives remarquables de ce matériau ne sont plus à démontrer. Le Bioglass® reste à l'heure actuelle un des matériaux bioactifs (induisant une réponse spécifique des cellules) les plus intéressants.

De nombreux développements ont été faits dans le domaine des verres bioactifs depuis leur découverte (M. Vallet-Regi et al., Eur. J. Inorg. Chem. 2003, 1029-1042), tels que l'incorporation de différents atomes ou l'incorporation de principes actifs. Les compositions des verres bioactifs ont été optimisées de façon à favoriser la prolifération des ostéoblastes et la formation de tissus osseux (WO 02/04606). L'incorporation d'argent a été proposée notamment pour conférer des propriétés antibactériennes aux verres bioactifs (WO 00/76486).

La demande WO 2009/027594 décrit, elle, un verre bioactif dans lequel le strontium est introduit en des quantités comprises entre 0,1 à 10% du poids total du verre bioactif. Les verres bioactifs sont utilisés dans la fabrication d'implant osseux (ANKE LISA METZE ET AL,BONE AND BIOMATERIALS FOR BONE TISSUE ENGINEERING, vol. 541, 2013-01-01, pages 31-39. Ces matériaux bioactifs présentent comme caractéristique d'être tout à la fois biocompatibles, capables de se lier spontanément aux tissus osseux, de promouvoir l'adhésion des cellules osseuses et, enfin d'être biorésorbables, étant progressivement remplacés par du tissu osseux néoformé à mesure que la repousse osseuse avance.

Cependant, malgré cet ensemble de caractéristiques très satisfaisantes, la fragilité de ces matériaux en limite les applications : en effet, bien que leur rigidité soit souvent supérieure à celles de l'os, leur manque de souplesse et de ténacité font que les matériaux bioactifs ne peuvent être implantés en des sites mécaniquement chargés.

Pour pallier ce défaut, une solution ingénieuse est de s'inspirer de la structure particulière du tissu osseux. Complexe, celle-ci consiste principalement en une trame composite mêlant intimement une phase inorganique, le minéral osseux constitué de cristaux d'apatite (phosphate de calcium résorbable), à une phase organique, qui est majoritairement du collagène. De manière remarquable, une telle structure composite associe la rigidité initiale de la partie inorganique à la ténacité et à la souplesse naturelle des fibres de collagène. Pour obtenir des implants aux propriétés mécaniques proches du tissu osseux, une stratégie consiste donc à combiner matériaux bioactifs et polymères biodégradables au sein d'une même matrice composite ou hybride.

Pour le comblement de larges défauts osseux, les implants doivent avoir, en plus des caractéristiques précédentes, une morphologie spécifique : celle-ci s'inspire de l'os trabéculaire, à savoir une structure hautement poreuse constituée d'un réseau tridimensionnel de macropores interconnectés de plusieurs centaines de microns. En effet, dans le cas de larges défauts osseux, les cellules de l'os ont besoin d'une matrice "support" extracellulaire capable de guider et de stimuler l'adhésion, la prolifération, la différentiation cellulaire, tout en étant compatible avec les processus de vascularisation et d'invasion tissulaire.

Une telle structure macroporeuse est également requise pour les nouvelles applications envisagées en ingénierie tissulaire de l'os : il s'agit, à partir de cellules prélevées chez le patient, de fabriquer en laboratoire du tissu osseux nouveau que l'on pourra ré-implanter a posteriori chez le patient. Pour être conduite de façon optimale, cette culture de tissu doit là aussi reposer sur des supports tridimensionnels poreux permettant une bonne adhésion cellulaire, la différentiation en cellules matures ainsi que la fabrication du tissu et en particulier la biominéralisation.

En résumé, si de nombreux matériaux et formulations ont été développés pour le comblement des pertes osseuses, aucun ne répond totalement au cahier des charges décrivant l'implant idéal, à savoir :
- être biocompatible ;
- être bioactif : induire spontanément la formation d'un lien interfacial fort avec les tissus osseux, promouvoir l'adhésion et l'activité cellulaire ;
- être biorésorbable ;
- avoir une morphologie adéquate basée sur une matrice tridimensionnelle de macropores interconnectés ;
- avoir une bonne tenue mécanique ;
- être dérivé d'un procédé de fabrication permettant une mise en forme facile et suffisamment souple pour s'adapter à de nombreuses géométries de défauts.

Par morphologie adéquate basée sur une matrice tridimensionnelle de macropores interconnectés, on entend que la taille, la forme et la distribution des pores ainsi que la taille des interconnexions entre ces pores doivent être contrôlées.

L'invention a pour but de proposer un matériau qui répond parfaitement à tous ces critères et qui peut être fabriqué par un procédé qui permet la réalisation d'architectures poreuses composées d'une partie inorganique et d'une partie organique, au contraire des procédés de l'art antérieur.

A cet effet, l'invention propose un procédé de fabrication d'un implant pour le comblement de défauts osseux, la régénération osseuse et l'ingénierie tissulaire de l'os en un matériau selon l'invention, caractérisé en ce qu'il comprend les étapes suivantes :
a) sélection d'un verre bioactif M à base de SiO₂ et CaO, contenant optionnellement P₂O₅ et/ou optionnellement dopé au strontium,
b) sélection d'un polymère biodégradable P qui est soluble dans un solvant S1 et insoluble dans au moins un solvant S,
c) sélection de microsphères d'un agent porogène A ayant des diamètres et des tailles correspondants aux diamètres et tailles recherchés des pores dans un matériau d'implant, le matériau de cet agent porogène A étant un polymère insoluble dans le solvant S1 et soluble dans le au moins un solvant S,
le au moins un solvant S dans lequel le polymère biodégradable P est insoluble et le au moins un solvant S dans lequel le matériau de l'agent porogène A est soluble étant identiques,
d) introduction d'au moins 60% en volume, de préférence d'au moins 70% (et moins de 100%) par rapport au volume total du mélange polymère biodégradable P- agent porogène A introduit dans le moule, de microsphères de l'agent porogène A dans un moule ayant la forme et la taille recherchées pour l'implant, ces microsphères formant un empilement compact correspondant à la forme et à la taille des pores à obtenir dans le matériau d'implant,
e) introduction du polymère biodégradable P dans le moule,
f) gélification du mélange obtenu à l'étape e) dans le moule,
g) démoulage du mélange obtenu à l'étape f),
h) élimination de l'agent porogène par lavage avec le au moins un solvant S,
i) réticulation du mélange obtenu à l'étape g),
j) revêtement du mélange obtenu à l'étape i) avec le verre bioactif M ou avec un matériau hybride H formé d'un polymère biodégradable identique ou différent du polymère biodégradable P et du verre bioactif M.

Dans un premier mode de mise en oeuvre du procédé de l'invention, l'étape j) est mise en oeuvre par imprégnation du mélange obtenu à l'étape i) avec une suspension, dans un solvant, contenant des particules du verre bioactif M ou du matériau hybride H, et évaporation du solvant.

Dans un second mode de mise en oeuvre du procédé de l'invention, l'étape j) est une étape de revêtement du mélange obtenu à l'étape i) soit avec le verre bioactif M, soit avec le matériau hybride H, et est mise en oeuvre par immersion du mélange obtenu à l'étape i) soit dans un sol contenant les précurseurs alcoxydes du verre bioactif M, pour un revêtement uniquement avec le verre bioactif M, soit dans un sol du matériau hybride, ou dans un sol des précurseurs alcoxydes du verre bioactif M et du polymère biodégradable du matériau hybride H, pour un revêtement avec le matériau hybride H, suivie d'une étape de gélification.

Dans tous les modes de mise en oeuvre du procédé de l'invention, le polymère biodégradable P est choisi parmi :
- les polymères biodégradables solubles dans au moins un solvant S1 et insolubles dans au moins un solvant S choisis parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique) (PLA),
- les polymères synthétiques biodégradables, de préférence un polyéthylène glycol, ou le poly(caprolactone)
- les protéines, de préférence la gélatine ou le collagène,
et le matériau de l'agent porogène A est choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine, le naphtalène et l'acrylonitrile butadiène styrène (ABS),
le matériau de l'agent porogène A étant différent du polymère biodégradable P.

Plus préférablement, le polymère biodégradable P est un polymère d'origine naturelle ou un polymère synthétique biodégradable ou un polyester biorésorbable.

Lorsque le polymère biodégradable P est revêtu d'un matériau hybride, de préférence, le rapport en poids polymère biodégradable / verre bioactif M dans ce matériau hybride est compris entre 10/90 et 90/10, bornes incluses, de préférence entre 20/80 et 80/20, bornes incluses, préférablement entre 30/70 et 70/30 bornes incluses.

Le plus préférablement, le matériau hybride est constitué de 70% en masse de polymère biodégradable et 30% en masse de verre bioactif.

Le polymère du matériau hybride du revêtement peut être identique ou différent du polymère biodégradable P.

Lorsque le polymère biodégradable P est recouvert du verre bioactif M seul, alors, de préférence le rapport en masse polymère biodégradable P / verre bioactif M est de préférence compris entre 50/50 et 90/10, de préférence entre 60/40 et 80/20.

Egalement de préférence, le verre bioactif M est un verre à base de SiO₂ et de CaO, le polymère biodégradable P est la gélatine, le matériau des microsphères d'agent porogène A est le polyméthacrylate de méthyle et le solvant S est l'acétone.

Cependant, le verre bioactif préféré est un verre constitué de 75% en masse de SiO₂ et 25% en masse de CaO ou un verre constitué de 75% en masse de SiO₂, 20% en masse de CaO et 5% en masse de SrO.

Le procédé de l'invention peut comprendre, de plus, une étape d'introduction d'un agent de couplage, de préférence un composé organoalcoxysilane, plus préférablement du 3-glycidoxypropyltriméthoxysilane (GPTMS) et encore plus préférablement du 3-glycidoxypropyltriéthoxysilane (GPTES) à l'étape e).

Il peut également comprendre, de plus, après l'étape d), et avant l'étape e), une étape d'agrandissement des interconnexions, par infiltration d'un solvant du matériau de l'agent porogène A, dans l'empilement des microsphères d'agent porogène A et/ou par chauffage de cet empilement.

L'invention propose encore un matériau d'implant, pour le comblement de défauts osseux, la régénération osseuse et l'ingénierie tissulaire de l'os, obtenu par le procédé selon l'invention,
caractérisé en ce qu'il comprend :
- un verre bioactif M à base de SiO₂ et CaO, optionnellement contenant P₂O₅ et/ou optionnellement dopé au strontium, et
- un polymère biodégradable P soluble dans au moins un solvant S1 choisi parmi :
   - les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
   - les polyesters biorésorbables, de préférence l'alcool polyvinylique ou le poly(acide lactique),
   - les polymères synthétiques biodégradables, de préférence un polyéthylène glycol, ou le poly(caprolactone), et
   - les protéines, de préférence la gélatine ou le collagène,
   et en ce que il consiste en une matrice comprenant au moins le polymère biodégradable P recouvert du verre bioactif M ou d'un matériau hybride H formé du verre bioactif M et d'un polymère biodégradable identique ou différent du polymère biodégradable P, cette matrice ayant au moins 70 % en nombre de pores ayant au moins une interconnexion avec un autre pore et la forme de sphères ou de polyèdres s'inscrivant dans une sphère, le diamètre des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions entre les pores ayant leur plus petite dimension comprise entre 25 µm et 250 µm, bornes incluses.

L'invention propose enfin un implant pour le comblement de défauts osseux, la régénération osseuse et l'ingénierie tissulaire de l'os, caractérisé en ce qu'il comprend un matériau selon l'invention ou obtenu par le procédé de fabrication d'un implant selon l'invention.

L'invention sera mieux comprise et d'autres caractéristiques et avantages de celle-ci apparaîtront plus clairement à la lecture de la description explicative qui suit et qui est faite en référence aux figures annexées dans lesquelles :

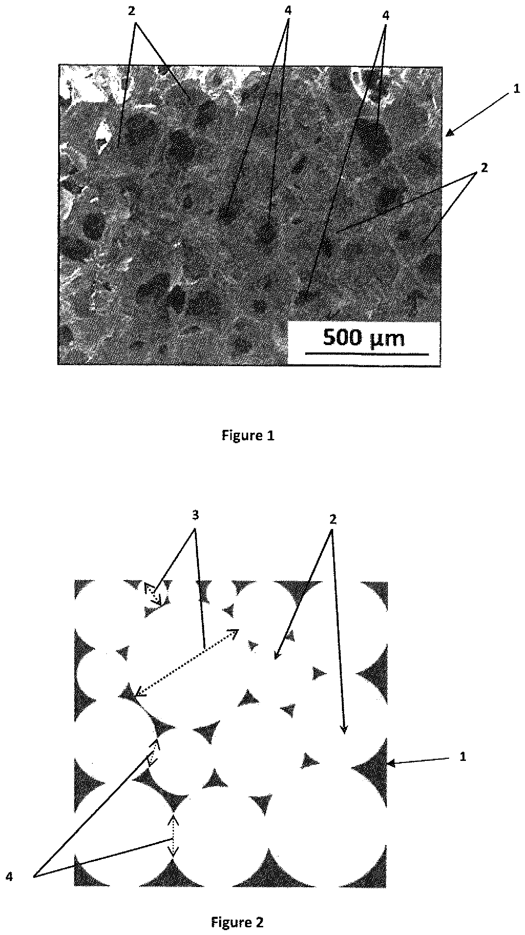

- la figure 1 représente une vue en coupe prise au microscope électronique à balayage de l'implant de l'invention obtenu à l'exemple 1, à un grandissement de x70,
- la figure 2 est une représentation schématique du matériau d'implant selon l'invention,

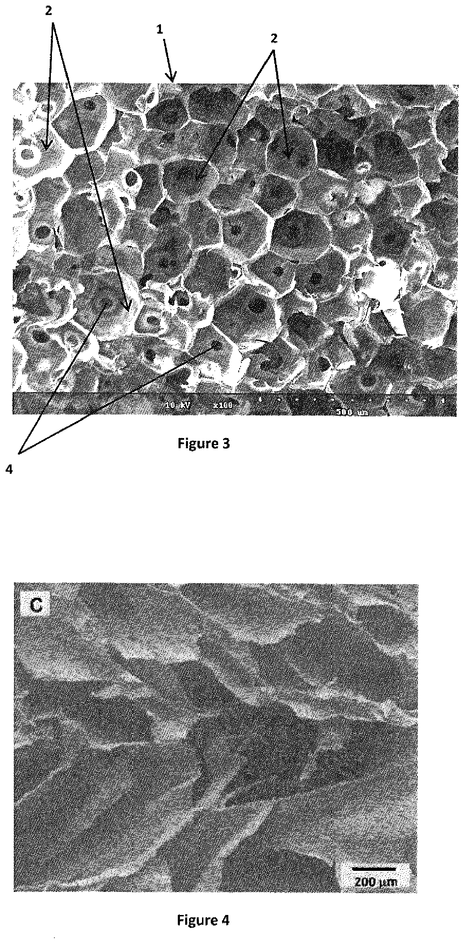

- la figure 3 représente une photographie prise au microscope électronique à balayage d'un implant de l'invention dont la matrice est constituée de gélatine recouverte d'un verre bioactif, à un grandissement de ×100,
- la figure 4 représente une photographie prise au microscope électronique à balayage, à un grandissement de x50 d'une coupe d'un matériau d'implant de l'art antérieur préparé par un procédé de lyophilisation, décrit dans Kim et al. "Hydroxyapatite and gelatin composite foams processed via novel freeze-drying and crosslinking for use as temporary hard tissue scaffolds" J Biomed Mater Res 72A: 136-145, 2005

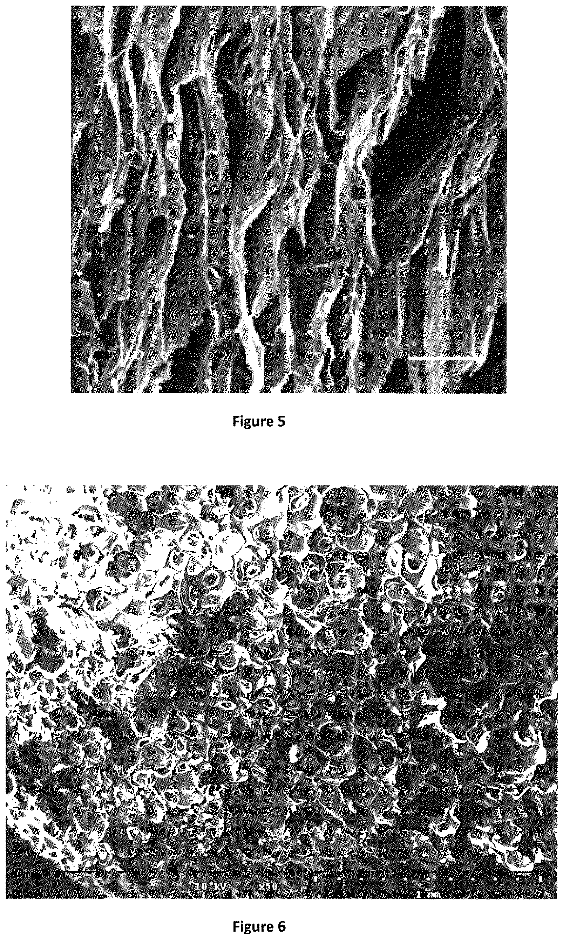

- la figure 5 représente une photographie prise au microscope électronique à balayage à un grandissement de ×200, d'un matériau d'implant de l'art antérieur préparé par un procédé de séparation de phases induite thermiquement, décrit dans Blaker et al. "Mechanical properties of highly porous PDLLA/Bioglass® composite foams as scaffolds for bone tissue engineering" Acta Biomater 2005, 1, 643-52,
- la figure 6 représente une photographie prise au microscope électronique à balayage à un grandissement de x50, d'un matériau d'implant hybride de rapport massique gélatine/verre 70/30, obtenu par un procédé comprenant une étape d'augmentation de la taille des interconnexions entre pores par infiltration avec un mélange acétone-éthanol à 30% en volume d'acétone, par rapport au volume total du mélange pendant 5 minutes par infiltration avec le mélange dans l'empilement des microsphères d'agent porogène, seul,

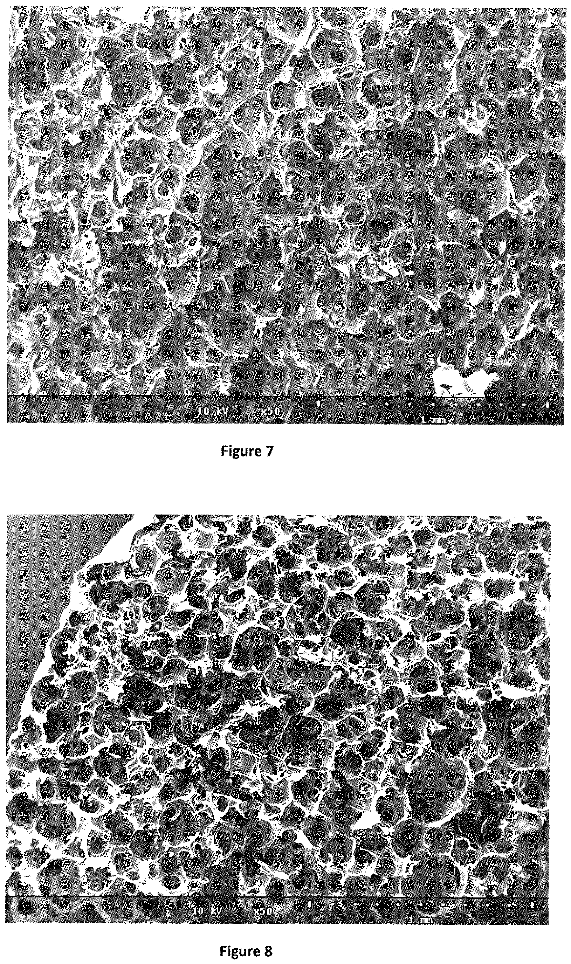

- la figure 7 est une photographie prise au microscope électronique à balayage à un grandissement de x50 du même matériau d'implant que représenté en figure 6 mais après infiltration avec un mélange acétone-éthanol à 30% en volume d'acétone, par rapport au volume total du mélange, pendant 15 minutes, avec le mélange dans l'empilement des microsphères d'agent porogène, seul,
- la figure 8 est une photographie prise au microscope électronique à balayage à un grandissement de ×50 du matériau représenté en figure 6 et en figure 7 obtenu par le procédé de l'invention après augmentation de la taille des interconnexions entre pores par infiltration du mélange acétone-éthanol à 30% en volume d'acétone, par rapport au volume total du mélange, pendant 30 minutes, avec le mélange dans l'empilement des microsphères d'agent porogène, seul,

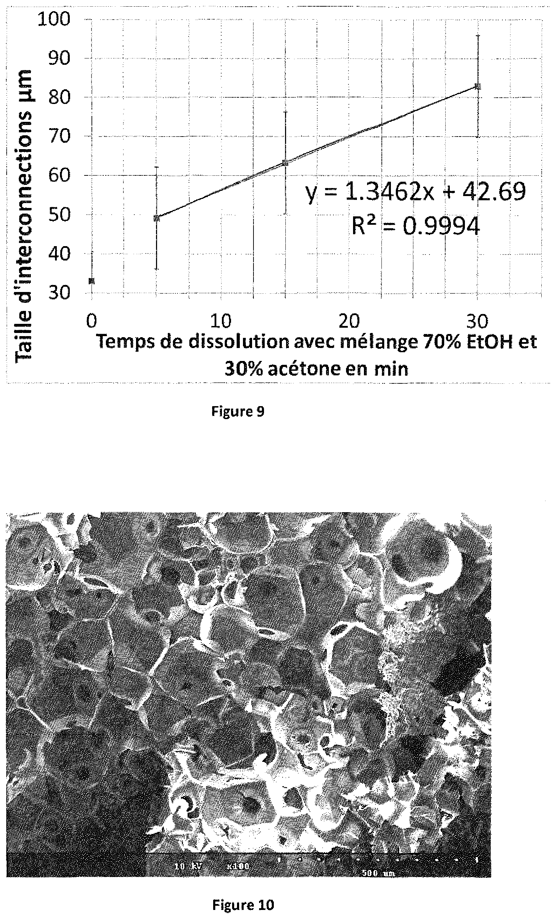

- la figure 9 est une courbe représentant l'augmentation de la taille des interconnexions entre pores par infiltration avec un mélange acétone-éthanol à 30% en volume d'acétone, par rapport au volume total du mélange, en fonction de ce temps d'infiltration,
- la figure 10 est une photographie prise au microscope électronique à balayage à un grandissement de ×100 d'un matériau d'implant hybride constitué de 70% de gélatine et 30 % de verre, en masse, selon l'invention obtenu par le procédé de l'invention après augmentation de la taille des interconnexions des pores par chauffage de l'empilement des microsphères d'agent porogène, seul, à 125°C pendant 15 minutes, sous air,

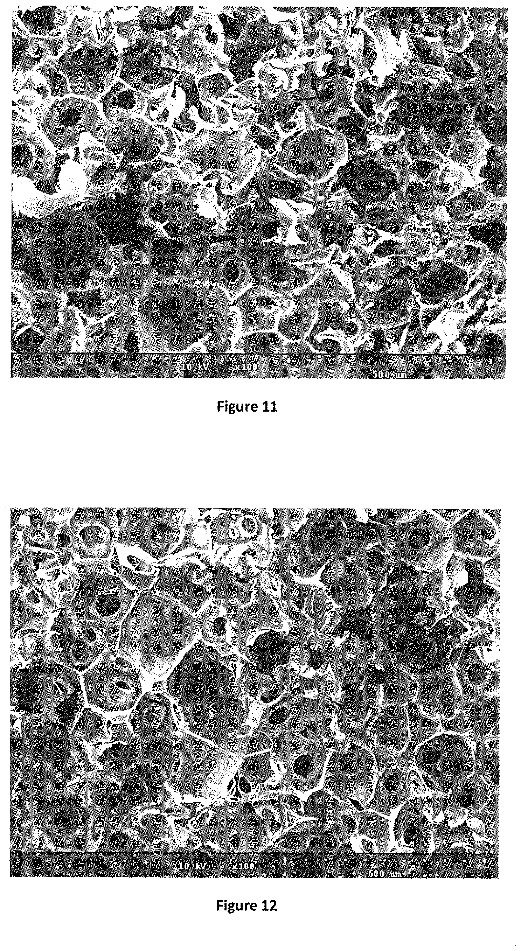

- la figure 11 est une photographie prise au microscope électronique à balayage à un grandissement de ×104 du matériau d'implant représenté en figure 10 mais après augmentation de la taille des interconnexions de pores par chauffage à 125°C pendant 1 heure de l'empilement de microsphères d'agent porogène, seul, avant infiltration avec le matériau hybride constitué de 70% de gélatine et 30 % de verre, en masse,
- la figure 12 est une photographie prise au microscope électronique à balayage à un grandissement de ×100 de la même composition de matériau d'implant selon l'invention que représenté en figure 10 et 11, obtenu par un procédé après augmentation de la taille des interconnexions de pores par chauffage à 125°C de l'empilement de microsphères d'agent porogène, seul, pendant 2 heures, et

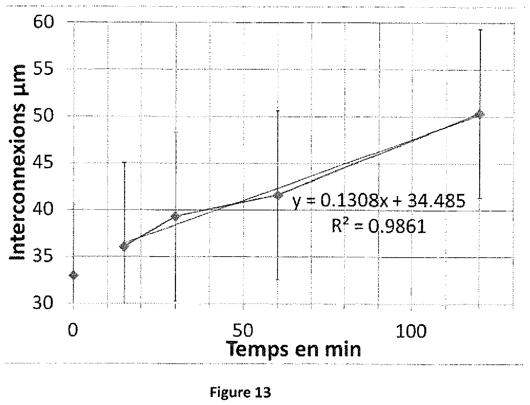

- la figure 13 représente la courbe montrant l'évolution de la taille des interconnexions entre pores en fonction du temps de chauffage à 125°C de l'empilement de microsphères d'agent porogène, seul.

Dans ce qui précède et ce qui suit, les termes suivants ont les définitions suivantes :
- "interconnexion(s) entre pores" : ouverture(s) permettant le passage d'un pore à l'autre,
- "milieu aqueux" ou "solvant aqueux" : tout milieu liquide contenant de l'eau, ou eau seule,
- "biodégradable" : dégradable dans un liquide physiologique, par exemple une solution saline tamponnée (SBF),
- "biorésorbable" : éliminable dans un milieu physiologique contenant des cellules biologiques,
- "diamètre moyen arithmétique de l'ensemble des pores" : somme des diamètres des pores / nombre de pores,
- "pore sphérique" ou "sphère" : pore ou sphère dont le rapport du plus petit diamètre sur le plus grand diamètre est de 0,9 ± 0,1,
- "polyèdre s'inscrivant dans une sphère" : polyèdre s'inscrivant dans une sphère ayant en tous points le même diamètre, les différences entre les différents diamètres du polyèdre s'inscrivant dans cette sphère étant d'au plus ± 15% du diamètre de la sphère dans laquelle ils s'inscrivent,
- "empilement compact de microsphères d'agent porogène A" : empilement de microsphères d'agent porogène A dans lequel au moins 60% en nombre, plus préférablement au moins 70% en nombre, de microsphères sont en contact les unes avec les autres, en l'absence du polymère biodégradable P et restent en contact les unes avec les autres lorsque le mélange polymère biodégradable P-agent porogène A est dans le moule.

On peut obtenir un tel empilement compact de microsphères d'agent porogène A par centrifugation du mélange microsphères d'agent porogène A-polymère biodégradable P ou encore en appliquant une pression négative (vide) ou positive (supérieure à la pression atmosphérique) sur le mélange microsphères d'agent porogène A-polymère biodégradable P introduit dans le moule, avant et pendant la gélification de ce mélange.

Le matériau d'implant pour le comblement de défauts osseux, la régénération osseuse et l'ingénierie tissulaire de l'os de l'invention sera décrit en relation avec les figures 1 et 2.

Comme on le voit en figures 1 à 3, le matériau d'implant de l'invention comprend une matrice, notée 1 en figures 1 et 2, en un matériau qui comprend une partie organique et une partie inorganique.

Ce matériau est biocompatible, bioactif, biorésorbable et comme on le voit sur les figures 1 à 3, il a une morphologie très régulière, en termes de distribution de pores, notés 2 en figures 1 à 3, en termes de forme de pores, au contraire des matériaux de l'art antérieur qui ont une distribution, une taille et une forme de pores anarchiques, comme on peut le voir en figures 4 et 5 qui représentent respectivement des photographies prises au microscope électronique à balayage de matériaux d'implant obtenus par un procédé de lyophilisation (figure 4) et un procédé de séparation de phases induite thermiquement (figure 5).

En particulier, ce matériau a des pores en forme de sphères dont le diamètre, noté 3 en figure 2, est de préférence identique en tous points, tel que le rapport du plus petit diamètre au plus grand diamètre est de 0,9 ± 0,1 ou encore en forme de polyèdres s'inscrivant dans une telle sphère, les différences entre les diamètres en différents points du polyèdre s'inscrivant dans cette sphère étant d'au plus plus ou moins 15 % du diamètre de la sphère dans laquelle ils s'inscrivent.

Au moins 70 % en nombre des pores du matériau d'implant de l'invention ont ces formes.

Les matériaux d'implant de l'invention peuvent avoir des tailles de pores se situant dans un très large intervalle de 100 µm à 900 µm, de préférence 200 µm à 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant en association avec des interconnexions, notées 4 en figures 1 à 3, entre pores dont la plus petite dimension est comprise entre 25 µm et 250 µm, bornes incluses.

Ainsi, une telle forme et distribution de tailles de pores ainsi que de telles tailles d'interconnexions entre pores sont très favorables à la conduction des cellules, à la repousse osseuse et à l'invasion tissulaire comme cela a été démontré par Karageorgiou et al., "Porosity of 3D biomaterial scaffolds and osteogenesis". Biomaterials 2005, 26, (27), 5474-5491.

Cependant, dans le cas de cet article, de telles formes de pores avaient été obtenues sur un implant entièrement en céramique bioactive, c'est-à-dire en un phosphate de calcium (hydroxyapatite).

Or, de telles céramiques ont l'inconvénient de ne pas présenter la flexibilité requise pour un implant osseux. Leur procédé de fabrication ne peut pas être appliqué à un matériau comprenant une partie organique, comme celui de l'invention, car il implique une étape de frittage à des températures de l'ordre de 800°C, auxquelles la partie organique se désintègre.

De telles distributions de tailles ne sont jamais atteintes dans les matériaux d'implants comprenant une partie organique et une partie inorganique issus des procédés de l'art antérieur pour lesquels les pores ont des tailles généralement bien inférieures à 200 µm avec des interconnexions de tailles bien inférieures.

Il existe bien des matériaux d'implants issus des procédés de moussage mais ceux-ci ont alors des distributions de tailles de pores et d'interconnexions très larges, non contrôlées, la porosité pouvant même atteindre le millimètre, ce qui est n'est pas favorable à la tenue mécanique de l'implant.

WO 2013/023064 décrit deux procédés pour obtenir une matrice composite ayant des pores dont la taille permet l'infiltration des cellules et la croissance interne d'os. Dans le premier procédé, on obtient un matériau fibreux (qui n'a donc pas de pores sphériques, à distribution de tailles contrôlée). Dans le deuxième procédé qui est un procédé de moulage par solvant, la porosité peut être augmentée par ajout d'un agent porogène. Mais l'exemple 1B décrivant ce procédé, lorsque reproduit n'a pas permis d'obtenir un implant, comme cela est montré à l'exemple comparatif donné dans ce qui suit.

Comme on le verra par la suite, grâce au procédé de fabrication des implants de l'invention, il est possible de contrôler la dispersion du jeu de tailles de pores et d'interconnexions de la matrice, ce qui n'était pas possible dans les procédés de l'art antérieur où la porosité générée est distribuée de manière aléatoire dans leurs intervalles respectifs.

La matrice 1 est constituée d'une phase organique et d'une phase inorganique.

La phase inorganique est un verre bioactif M.

Les céramiques bioactives et verres bioactifs sont bien connus de l'homme du métier et sont décrits en particulier dans L.L. Hench et al., J. Biomed. Mater. Res. 1971, 2, 117-141 ; L.L. Hench et al., J. Biomed. Mater. Res. 1973, 7, 25-42 pour les céramiques bioactives et dans M. Vallet-Regi et al., Eur. J. Inorg. Chem. 2003, 1029-1042 et WO 02/04606, WO 00/76486 et WO 2009/027594, en particulier. Dans l'invention, on utilise uniquement un verre bioactif

La partie organique du matériau d'implant de l'invention est un polymère biodégradable P choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone), et
- les protéines, de préférence la gélatine ou le collagène.

Bien entendu, lorsque l'agent porogène A est en poly(acide polylactique) ou en poly(caprolactone), le polymère biodégradable P sera en un polymère différent.

La matrice 1 peut consister en le verre bioactif M et le polymère biodégradable P qui forment un matériau composite, c'est-à-dire que les deux phases verre bioactif M et polymère biodégradable P coexistent dans l'architecture de la matrice. Ce n'est pas le cas dans l'invention.

La matrice 1 peut également être constituée du verre bioactif M et du polymère biodégradable P qui forment un matériau hybride, c'est-à-dire formant une seule phase. Dans ce cas, le matériau hybride est obtenu en formant un sol contenant tous les précurseurs alcoxydes du verre bioactif, en ajoutant le polymère biodégradable voulu pour le matériau hybride H dans ce sol et en gélifiant la solution ainsi obtenue par une succession de réactions de polymérisation (polymérisation sol-gel de la phase inorganique) (condensation des alcoxydes). On obtient alors un mélange hybride associant intimement la phase minérale et la phase organique. Ce n'est pas le cas dans l'invention.

Le matériau hybride se distingue donc du matériau composite par une intégration intime entre les deux phases organique et inorganique, ces deux phases étant indiscernables (sauf à l'échelle moléculaire) dans le cas d'un mélange hybride. Ce qui, encore une fois, n'est pas le cas dans l'invention.

En effet, dans l'invention, la matrice 1 est formée du seul polymère biodégradable P, polymère qui est recouvert du verre bioactif M, par exemple par imprégnation de la matrice 1 en polymère biodégradable P dans une suspension du verre bioactif M ou lorsque la matrice 1 est recouverte du matériau hybride H en immergeant la matrice 1 uniquement formée du polymère biodégradable P dans un sol du matériau hybride, ou dans un sol des précurseurs alcoxydes du verre bioactif M et du polymère biodégradable du matériau hybride H.

Dans les deux cas, la matrice 1 sera ensuite séchée pour permettre le dépôt des particules de verre bioactif M ou la gélification du sol selon le cas.

Le matériau d'implant de l'invention est obtenu par un procédé faisant appel à un agent porogène A qui est constitué de microsphères en un polymère soluble dans au moins un solvant S dans lequel le polymère biodégradable P n'est, lui, pas soluble.

Ainsi, le procédé de l'invention consiste à empiler des microsphères d'agent porogène A en un matériau polymère, différent du polymère biodégradable P, dans un moule ayant la forme et la taille correspondant à la géométrie du défaut osseux à combler ou du défaut où la régénération osseuse est voulue. Cet empilement est un empilement compact tel que défini précédemment.

Ces microsphères d'agent porogène A permettent d'obtenir au final des pores dont la taille et la distribution correspondront en négatif à l'empilement de microsphères d'agent porogène A initialement réalisé.

De plus, au moins 70% en nombre de pores formés auront la forme de sphères parfaites, c'est-à-dire auront en tous points un diamètre égal ou auront un rapport du plus petit diamètre au plus grand diamètre de 0,9 ± 0,1 ou, pour les plus grands pores, auront la forme d'un polyèdre s'inscrivant dans une sphère ayant en tous points le même diamètre, les différences entre les diamètres en différents points du polyèdre s'inscrivant dans cette sphère étant d'au plus plus ou moins 15 % du diamètre de la sphère dans laquelle ils s'inscrivent.

En effet, le matériau destiné à constituer la matrice 1 sera ensuite infiltré dans l'empilement des billes de microsphères d'agents porogènes A, puis solidifié pour pouvoir être démoulé sans changer la forme et la taille de l'empilement de l'implant voulu. L'agent porogène A sera alors éliminé permettant l'obtention du matériau d'implant de l'invention.

Comme on le voit, ce procédé n'utilise aucun traitement thermique à haute température pour fritter le verre bioactif M, la seule mise en température nécessaire étant la température d'évaporation du solvant utilisé.

Dans le procédé de l'invention, la matrice 1 est constituée uniquement du polymère biodégradable P et est ensuite recouverte soit du verre bioactif M, soit d'un matériau hybride constitué d'un polymère biodégradable et du verre bioactif M.

Comme cela apparaîtra clairement, l'invention réside sur la judicieuse association du choix de trois matériaux : le matériau constituant le polymère biodégradable P, le matériau constituant l'agent porogène A et le solvant S de l'agent porogène A qui ne doit pas dissoudre le polymère biodégradable P.

Le matériau du polymère biodégradable P qui fait partie du matériau d'implant doit être un polymère biocompatible.

Le matériau de l'agent porogène A, doit être, lui, un matériau, par exemple un polymère, dont le solvant est un non-solvant du polymère biodégradable P.

On comprend alors que le choix de l'un des trois éléments du trio "polymère biodégradable P-agent porogène A-solvant S du porogène" ne peut se faire indépendamment du choix des autres.

Les polymères biodégradables P doivent être solubles dans au moins un solvant S1 et insolubles dans au moins un solvant S.

Le solvant S1 peut être de l'eau, un milieu aqueux ou un solvant organique. Il en est de même du solvant S.

Parmi les polymères biodégradables P utilisables se trouvent :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone), et
- les protéines, de préférence la gélatine ou le collagène.

Tous ces polymères sont solubles dans au moins un solvant S1 et insoluble dans au moins un solvant S.

Dans le cas des polyéthylènes glycols, ces polymères sont solubles dans l'eau et dans de nombreux solvants organiques excepté le diéthyl éther et l'hexane.

Le matériau constituant l'agent porogène A doit être soluble dans le au moins un solvant S dans lequel le polymère biodégradable P est insoluble.

Des exemples de tels matériaux sont les polyméthacrylates d'alkyle en C₁ à C₄, de préférence le polyméthacrylate de méthyle (PMMA) ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène et l'acrylonitrile butadiène styrène (ABS)

Des exemples de polymères entrant dans la composition du matériau hybride H destiné à revêtir le polymère biodégradable P dans les modes de mise en oeuvre du procédé de l'invention sont les mêmes que ceux cités pour le polymère biodégradable P. Mais ce polymère et le polymère biodégradable P peuvent être identiques ou différents l'un de l'autre.

Le matériau de l'agent porogène A doit également être différent du polymère biodégradable P.

Dans tous les cas, le solvant du matériau de l'agent porogène A ne devra pas être un solvant pour le matériau choisi pour servir de polymère biodégradable P.

Les solvants S sont en particulier l'acétone, l'éthanol, le chloroforme, le dichlorométhane, l'hexane, le cyclohexane, le benzène, l'éther diéthylique, l'hexafluoroisopropanol.

Dans l'invention, de préférence, le polymère biodégradable P sera la gélatine, les microsphères d'agent porogène A seront en polyméthacrylate de méthyle, le solvant S sera l'acétone et le solvant S1 sera l'eau.

Dans le procédé de fabrication de matériau d'implant de l'invention, l'introduction des microsphères dans le moule peut être faite avant l'introduction du polymère biodégradable P.

Cependant, il est également possible de d'abord introduire le polymère biodégradable P dans le moule, et d'y verser ensuite les microsphères d'agent porogène A.

Pour obtenir un matériau dont au moins 70% en nombre de pores, ont au moins une interconnexion avec un autre pore, la quantité d'agent porogène A introduite dans le polymère biodégradable P doit représenter au moins 60%, de préférence au moins 70%, en volume, du volume total du mélange polymère biodégradable P-agent porogène A introduit dans le moule.

La taille des interconnexions est liée à la taille du point de contact entre sphères porogènes dans l'empilement de sphères réalisé. L'augmentation de la taille des interconnexions générées, à diamètre poreux constant, est possible moyennant l'ajout d'une étape consistant en une fusion partielle des sphères porogènes dans l'empilement initialement réalisé, de manière à augmenter la taille de leur point de contact.

Ce fusionnement peut se faire par infiltration d'un solvant du matériau de l'invention sur l'empilement de l'agent porogène A, ou bien par échauffement de l'empilement des microsphères d'agent porogène réalisé, ou bien des deux à la fois, de manière à réaliser la dissolution superficielle des sphères et permettre leur fusionnement partiel.

Les figures 6 à 8 montrent l'effet d'augmentation de la taille de ces interconnexions par infiltration d'un mélange acétone-éthanol à 30% en volume d'acétone, par rapport au volume total du mélange, après 15 min, 30 min et 1 heure d'infiltration.

Cette infiltration a lieu directement sur l'empilement de microsphères d'agent porogène, avant l'introduction du verre bioactif M et/ou du polymère biodégradable P.

La figure 9 représente cet effet d'augmentation sous forme de courbe.

Les figures 10 à 12 montrent l'effet d'augmentation de la taille de ces interconnexions par chauffage à 125°C, de l'empilement de microsphères d'agent porogène, avant l'introduction du verre bioactif M et/ou du polymère biodégradable P, pendant 15 min, 1 heure et 2 heures.

La figure 13 représente cet effet d'augmentation sous forme de courbe.

Afin de mieux faire comprendre l'invention, on va en décrire maintenant, à titre purement illustratif et non limitatif, plusieurs exemples de mise en oeuvre.

**Exemple 1 :** Fabrication d'un implant en un matériau composite gélatine, en tant que polymère biodégradable P, et un verre bioactif constitué de 75% de SiO₂ et 25% de CaO, en masse, par rapport à la masse totale du verre, en tant que verre bioactif M (non partie de l'invention).

La gélatine a été choisie en tant que matériau pour le polymère biodégradable P car c'est un biopolymère naturel, biocompatible, bon marché et facilement disponible. La gélatine est de plus dérivée du collagène naturellement présent dans les os. Elle est d'autre part déjà utilisée dans le cadre d'applications cliniques, pansements, adhésifs et encapsulations de substances pharmaceutiques.

Un verre bioactif a été choisi en raison de sa grande capacité à induire la minéralisation, la possibilité de façonner leurs propriétés texturales et morphologiques (porosité, taille et donc surface spécifique) à l'échelle nanométrique, la grande gamme de compositions bioactives qu'il est possible de formuler, en y ajoutant par exemple des éléments anti-inflammatoires, ou ostéoinducteurs, et enfin c'est la combinaison de leurs propriétés de bioactivité et de biorésorbabilité qui en font les biomatériaux le plus prometteurs pour la régénération osseuse, notamment par rapport aux phosphates de calcium (céramique bioactive), qui sont en général soit moins bioactifs, soit moins résorbables.

Les microsphères sont des microsphères en polyméthacrylate de méthyle. Ce matériau a été choisi car il peut être aisément dissous par de nombreux solvants.

De plus, si des résidus de polyméthacrylate de méthyle non éliminés venaient à subsister dans le matériau d'implant, la bonne biocompatibilité de ce polymère avec les tissus humains est une bonne garantie que l'implant ne présentera aucun risque de cytotoxicité.

L'agent porogène était sous la forme de particules sphériques, à savoir des billes de polyméthacrylate de méthyle.

Leurs diamètres peuvent être choisis entre plusieurs dizaines à plusieurs centaines de microns, suivant les applications. La porosité du matériau d'implant de l'invention qui sera finalement obtenu peut être contrôlée sur ces deux points ; premièrement le diamètre des pores qui sera obtenu dépend directement du diamètre des particules porogènes initiales. Il suffit donc d'ajuster la granulométrie des microsphères de polyméthacrylate de méthyle initiales en vue d'obtenir très simplement la porosité désirée. Deuxièmement la taille des interconnexions entre pores dépend directement de la taille de la zone de contact entre les billes polymères dans l'empilement initial. La taille de cette zone de contact peut être modifiée en faisant fusionner entre elles les particules de polymère initiales, au moyen d'un solvant, ou par un traitement thermique préliminaire. Cette procédure a déjà été décrite par Descamps et al., "Manufacture of macroporous beta-tricalcium phosphate bioceramics". Journal of the European Ceramic Society 2008, 28, (1), 149-157 et "Synthesis of macroporous beta-tricalcium phosphate with controlled porous architectural". Ceramics International 2008, 34, (5), 1131-1137.

Dans cet exemple, le polymère biodégradable et le verre bioactif ont été utilisés pour obtenir une matrice composite.

Ainsi, à cet exemple, la première étape consistait à placer dans un moule à la taille et à la forme recherchées pour l'implant, des particules d'agents porogènes constituées de microsphères de polyméthacrylate de méthyle.

Dans une deuxième étape, on a introduit la poudre de verre bioactif.

La granulométrie de la poudre de verre bioactif joue un rôle important dans l'obtention d'une matrice composite homogène. De préférence, la granulométrie de la poudre de verre bioactif doit être très inférieure à 50 µm. Idéalement, la taille des particules de poudre doit être de l'ordre du micromètre, ou même de l'ordre du nanomètre à quelques centaines de nanomètres. Une telle finesse peut être obtenue au moyen d'un broyeur planétaire à billes par exemple.

Dans une troisième étape, la gélatine, préalablement dissoute dans de l'eau tiède, est introduite. Le mélange composite est alors homogénéisé.

Dans une quatrième étape, le mélange obtenu à la troisième étape est gélifié pendant plusieurs heures, dans le moule, la déshydratation partielle de la gélatine assurant la prise du mélange.

Cette opération est menée à une température comprise entre 0°C et 60°C, inclus, afin de ne pas dégrader la matrice.

Dans une cinquième étape, les microsphères d'agent porogène en polyméthacrylate de méthyle sont éliminées par lavage à l'acétone.

L'acétone présente plusieurs intérêts : tout d'abord les billes de polyméthacrylate de méthyle sont facilement dissoutes dans l'acétone et la gélatine est, quant à elle, insoluble dans l'acétone.

L'acétone permet de plus de poursuivre, si nécessaire, la déshydratation de la gélatine.

Enfin, c'est un solvant d'usage très courant, relativement économique, particulièrement disponible, qui ne représente pas de risques sérieux de toxicité.

Après plusieurs étapes de lavage, l'empreinte initiale des microsphères de polyméthacrylate de méthyle est complètement éliminée et le matériau final est obtenu, sous la forme d'un bloc macroporeux bio-composite en verre bioactif/gélatine.

La biodégradabilité de ce matériau d'implant en milieu vivant et sa tenue mécanique peuvent de plus être ajustées facilement en réticulant la gélatine lors d'une ultime étape d'immersion dans une solution d'un agent réticulant comme par exemple la génipine, le carbodiimide, le glutaraldéhyde, le formaldéhyde.

Cependant, cette étape est optionnelle.

Les structures obtenues peuvent être lavées sans aucun dommage dans des bains d'éthanol, afin d'éliminer d'éventuels résidus indésirables, tels que des chlorures, de l'acétone, etc.

A cet exemple, on a obtenu un matériau d'implant comprenant 60 % en masse de verre bioactif et 40 % en masse de gélatine, par rapport à la masse totale de l'implant.

**Exemple 2 :** Fabrication d'un matériau d'implant selon l'invention avec une matrice en matériau hybride (non partie de l'invention).

On a débuté par l'étape d'empilement des microsphères d'agent porogène polyméthacrylate de méthyle dans un moule ayant la géométrie recherchée pour l'implant.

Dans une deuxième étape le mélange hybride a été versé dans le moule contenant l'empilement de billes.

Une centrifugation ou une infiltration sous pression ou une infiltration sous vide peuvent être utilisées pour aider le mélange hybride à remplir les interstices entre les microsphères de polyméthacrylate de méthyle.

Le matériau hybride a été obtenu par un procédé sol-gel.

Dans ce procédé, un sol contenant tous les précurseurs alcoxydes du verre bioactif est amené à gélifier par une succession de réactions de polymérisation.

Dans le cas du présent exemple, la gélatine (le polymère biodégradable P) a été ajoutée avant gélification du sol, de manière à obtenir un mélange hybride.

Pour la réalisation de matériau hybride, une difficulté majeure est que les traitements thermiques à haute et moyenne température, c'est-à-dire supérieurs à 150°C sont à proscrire.

Or, dans les procédés décrits dans l'art antérieur et notamment dans Lin, S. et al., "Nanostructure evolution and calcium distribution in sol-gel derived bioactive glass". Journal of Materials Chemistry 2009, 19, (9), 1276-1282, ces traitements thermiques sont indispensables pour l'obtention d'un réseau vitreux homogène, notamment pour l'incorporation du calcium au sein du réseau silicate.

L'utilisation d'un précurseur alcoxyde pour le calcium permet l'incorporation du calcium dans la phase inorganique sans traitement thermique.

Cependant la très grande réactivité des alcoxydes de calcium vis-à-vis des réactions d'hydrolyse/condensation en présence d'eau font que le sol obtenu est très instable, la polymérisation sol-gel ayant lieu extrêmement rapidement, ce qui a rendu impossible jusqu'à ce jour sa manipulation en vue de réaliser un implant poreux et n'a pas permis non plus une bonne incorporation du calcium dans le réseau silicate. Ainsi, les inventeurs ont découvert qu'en limitant au maximum l'introduction d'eau dans le sol et en utilisant un précurseur alcoxyde différent de celui utilisé dans la littérature (Ramila A. et al., "Synthesis routes for bioactive sol-gel glasses : alkoxides versus nitrates". Chemistry of Materials 2002, 14, (12), 542-548) (à savoir le méthoxyethoxyde de calcium), il est possible d'augmenter grandement la stabilité du sol. Les réactions d'hydrolyse/condensation sont alors suffisamment lentes pour permettre une incorporation homogène du calcium dans le réseau silicate, tout en restant suffisamment rapides pour permettre la polymérisation de la phase inorganique. Dans l'exemple, les précurseurs alcoxydes de silicium et calcium sont mélangés ensemble dans une solution alcoolique légèrement acidifiée. De préférence, les précurseurs alcoxydes sont le tétraéthoxysilane et l'éthoxyde de calcium. Ensuite la gélatine préalablement dissoute est ensuite ajoutée à ce mélange pour l'obtention d'un sol hybride. Les seuls apports d'eau se font par l'intermédiaire de l'acide et de la solution de gélatine : ceci est suffisant pour permettre les réactions d'hydrolyse/condensation tout en les limitant fortement de manière à avoir un sol stable et manipulable entre quelques minutes et quelques heures suivant les proportions des réactifs.

Le matériau d'implant est alors obtenu en appliquant les quatrième et cinquième étapes telles que réalisées à l'exemple 1.

Que ce soit lors de l'élaboration du mélange composite ou du mélange hybride, il peut être avantageux d'ajouter un agent couplant au mélange, tel qu'un organoalcoxysilane.

Par exemple, celui-ci peut être simplement ajouté à la solution aqueuse du polymère biodégradable P, ici la gélatine. Le rôle de l'agent couplant est de fonctionnaliser la gélatine, en vue de permettre l'établissement de liaisons covalentes avec la phase inorganique (réseau silicate du verre bioactif). Dans le cas d'un mélange composite, le couplage permet d'obtenir des particules de verre bioactif liées en surface à la gélatine. Dans le cas d'un mélange hybride, un véritable copolymère organo-minéral est obtenu. L'intérêt est de pouvoir maîtriser à façon la dégradabilité de l'implant composite ou hybride ainsi que sa tenue mécanique, en agissant simplement sur le degré d'affinité entre phases organique et inorganique.

Un exemple d'agent couplant utilisé avec succès dans l'invention est le GPTMS (3-glycidoxypropylmethoxysilane), qui est soluble dans une solution aqueuse de gélatine.

**Exemple 3 :** Fabrication d'un matériau d'implant selon l'invention à matrice polymère biodégradable P recouverte de verre bioactif

Dans cet exemple, l'agent porogène A était des micropshères de PMMA d'un diamètre de 200 à 400µm qui représentait 70% en volume du volume total du mélange introduit dans le moule.

On a ensuite procédé comme à l'exemple 2, sauf qu'à la deuxième étape, seule la gélatine a été introduite, et qu'après la cinquième étape d'élimination par lavage des microsphères de polyméthacrylate de méthyle, le polymère biodégradable P, ici la gélatine a été réticulée dans une solution de glutaraldéhyde.

Le solvant S1 ici est l'eau et le solvant S est l'acétone.

Ensuite, la matrice en polymère biodégradable P est immergée dans une suspension du verre bioactif M ou encore immergée dans un sol contenant tous les précurseurs alcoxydes du verre bioactif M.

Dans les deux cas, la matrice 1 est ensuite séchée pour permettre le dépôt des particules de verre bioactif M ou la gélification du sol selon le cas.

Le rapport en masse polymère biodégradable P/verre bioactif était de 70/30.

**Exemple 4:** Fabrication d'un matériau hybride (non partie de l'invention).

### Produits utilisés :

- Téraéthylorthosilicate TEOS
- Ethoxyde de Calcium Ca(OEt)₂
- 3-glycidoxypropyltrimethoxysilane GPTMS
- HCl 2M et HCl 10mM
- Ethanol absolu
- Gélatine type B
- Acétone

### Protocole :

1. Remplir un tube en polyéthylène de 32 mm de hauteur et 9 mm de diamètre avec des billes de PMMA sur une hauteur d'environ 10 mm.
2. Mélanger 7,80g de TEOS et 6,39g d'éthanol dans un flacon.
3. Agiter pendant 15 min à l'aide d'un agitateur magnétique.
4. Ajouter 1,35mL de HCl à 2M dans le mélange Ethanol + TEOS.
5. Agiter pendant 30 min.
6. Peser 6,39 g d'éthanol dans un autre flacon.
7. Ajouter 1,74g d'éthoxyde de calcium.
8. Agiter 15 min.
9. Ajouter le sol contenant le TEOS à la solution d'éthoxyde de calcium.
10. Agiter au moins 1 heure.
11. Dissoudre 1,26g de gélatine de type B et 0,63g de GPTMS dans 8,74g d'HCl 10mM dans un bain marie à 60°C.
12. Prélever 3g de sol de bioverre et ajouter 7g de sol de gélatine greffé GPTMS dans un flacon.
13. Agiter quelques minutes, avec un agitateur magnétique.
14. Ajouter le sol hybride sur les billes PMMA.
15. Centrifuger 1 min
16. Laisser gélifier à une température comprise entre 0°C et 60°C, au moins 24 heures
17. Démouler le bloc hybride obtenu.
18. Dissolution des billes PMMA dans un flacon rempli d'acétone en renouvelant l'acétone au bout de 24 heures. Cette opération est à réitérer 2 fois.
19. Récupérer le bloc poreux obtenu et le mettre à sécher à l'étuve à 60°C pendant 24 heures

**Exemple 5 :** Préparation d'implants poreux composites avec 60% de bioverre (75 % SiO₂- 25% CaO) et 40% de gélatine (% massiques) (non partie de l'invention)

### 1) Synthèse de la poudre de verre par voie sol-gel

13,48 mL d'eau et 13,48 mL d'éthanol sont mélangés à 2,25 mL d'HCl à 2N puis 13,94 mL de TEOS sont ajoutés. Après 30 minutes d'agitation 5,2637 g de Ca(NO₃)₂.4H₂O sont ajoutés. Le sol est agité pendant 1 heure, mis à l'étude à 60°C dans des conteneurs en téflon pendant 24h puis mis à l'air à 125°C durant 24h. La poudre ainsi obtenue est ensuite calcinée pendant 24 heures à 700°C (chauffage de 25 à 700°C effectué en 2 heures).

La poudre est alors broyée pendant 30 minutes puis tamisée pour ne conserver que la fraction inférieure à 50 µm.

### 2) Préparation du composite

De la poudre de gélatine de porc (type A) est ajoutée à de l'eau distillée chauffée à 35°C selon un rapport de 0,1 g/mL d'eau, le mélange est agité pendant 10 minutes. Parallèlement, une quantité de 0,025 g de poudre de verre est mélangée à 0,2 g de billes PMMA. 0,15 mL de solution de gélatine dans l'eau sont alors ajoutés, le mélange obtenu est versé dans un tube dans lequel il est compacté.

Après 1 jour de séchage à l'air ambiant, le cylindre de verre + billes + gélatine est sorti du moule et immergé dans de l'acétone pendant 6 heures sous agitation, l'acétone est alors renouvelé et la dissolution est laissée se poursuivre 24 heures, toujours sous agitation. Le bloc composite poreux verre-gélatine obtenu est alors rincé à l'acétone et séché à l'air ambiant.

**Exemple 6 :** Evaluation *in vitro* des implants obtenus aux exemples 1 à 5.

La bioactivité des matériaux d'implants obtenus aux exemples 1 à 3 a été évaluée *in vitro* en les immergeant dans une solution physiologique (SBF) ayant une composition ionique identique à celle du plasma sanguin (test ISO-23317).

On a alors vérifié la grande bioactivité caractéristique des verres bioactifs utilisés dans les matériaux d'implants : ces matériaux d'implants se révélant très prompts à induire la minéralisation au contact du milieu physiologique : dès 1 h d'interaction avec le milieu, une partie des ions calcium issus de la matrice vitreuse a migré en surface du composite, où des ions phosphates provenant du milieu physiologique ont été incorporés pour former une couche de phosphate de calcium d'une dizaine de microns d'épaisseur, qui revêt la surface des pores.

Ceci constitue la première étape du processus de bioactivité.

On a vérifié que par la suite cette couche de phosphate de calcium continue à croître pour former une couche apatitique analogue au minéral osseux.

La réticulation de la gélatine n'amoindrit pas la bioactivité de l'implant, mais permet d'augmenter sa résistance à la dissolution en milieu physiologique.

On a également noté que le milieu SBF est rapidement (dès 1 jour) épuisé en phosphore, et dans une moindre mesure en calcium, ces éléments étant incorporés en surface des implants et donc retranchés du milieu pour former une couche de phosphate de calcium biomimétique.

La réticulation de la gélatine n'altère aucunement la réactivité chimique des implants, mais présente l'avantage de permettre d'ajuster leur biodégradabilité en milieu vivant.

Ainsi, tous les matériaux fabriqués aux exemples 1 à 5 se révèlent prompts à induire la formation de minéral osseux au contact des fluides physiologiques.

Cependant, on note des différences entre ces matériaux.

Tout d'abord, les matériaux dans lesquels la gélatine a été réticulée ont une biodégradabilité ralentie, ce qui se manifeste par la mise en solution plus lente du silicium. La formation de phosphates de calcium est également ralentie.

Ensuite, on constate que la formation de phosphates de calcium à la surface du matériau est plus lente avec le matériau composite qu'avec le matériau constitué du polymère biodégradable P revêtu du verre bioactif M selon l'invention.

Ceci représente un avantage certain.

Avec ces deux matériaux, comme attendu, la formation des phosphates de calcium et en particulier d'apatite, ne se fait qu'en surface du matériau.

En contraste avec le matériau hybride, la formation de phosphates de calcium se fait non seulement en surface mais également dans la masse, ce qui représente un inconvénient lorsque le défaut osseux à combler nécessite une intégration plus lente.

### Exemple 7:

On a procédé comme à l'exemple 3 sauf que la matrice en polymère biodégradable P a été immergée dans un sol des précurseurs alcoxydes du verre bioactif M et du polymère biodégradable du matériau hybride H obtenu à l'étape 13 de l'exemple 4.

Le matériau de l'invention ainsi obtenu a été évalué *in vitro* comme décrit à l'exemple 6.

Ce matériau présente l'avantage d'une croissance très rapide de phosphates de calcium non seulement en surface mais également dans le volume du matériau de l'invention.

**Exemple comparatif :** Fabrication d'un implant selon WO 2013/023064.

De la poudre de gélatine de porc (type A) est ajoutée à de l'eau distillée chauffée à 35°C selon un rapport de 0,1 g/mL d'eau, le mélange est agité pendant 10 minutes. Une quantité de 0,75 g de poudre de verre est mélangée à 57,38 g de particules de NaCl, puis 4,5 mL de la solution de gélatine sont alors ajoutés, de sorte à ce que le volume de porogène représente 90 % du volume total du mélange, comme indiqué dans WO 2013/023064. Le mélange obtenu est versé dans un tube. Le moule et son mélange subissent alors une congélation, puis une lyophilisation sous vide pendant une journée. Après lyophilisation, le bloc composite est démoulé et immergé dans de l'eau distillée afin de dissoudre le porogène (NaCl).

Malheureusement, la quantité de porogène (90% du volume total) s'est avérée beaucoup trop importante relativement à la quantité de mélange composite : la dissolution du porogène a entraîné la destruction immédiate de la structure composite, et aucun implant n'a pu être obtenu par ce protocole d'élaboration.

### Caractérisation de la sphéricité des macropores obtenus

La voie de synthèse proposée permet l'obtention de pores sphériques. En effet, en mesurant deux diamètres perpendiculaires pour chaque pore, le rapport du plus petit diamètre sur le plus grand diamètre est en moyenne de **0,9 ± 0,1.**

Ainsi, il apparaît clairement que grâce au procédé de l'invention, des implants ayant toutes les propriétés de porosité, en termes de tailles de pores, sphéricité de ces pores, distribution de cette taille des pores dans une très large gamme comprise entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant, peuvent être obtenus, en association avec des interconnexions entre pores dont la plus petite dimension est comprise entre 25 microns et 250 micromètres inclus, ce qui n'avait jamais été obtenu auparavant.

## Revendications

1. Procédé de fabrication d'un implant pour le comblement de défauts osseux, la régénération osseuse et l'ingénierie tissulaire de l'os, **caractérisé en ce qu'**il comprend les étapes suivantes :
a) sélection d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S,
c) sélection de microsphères d'un agent porogène A ayant des diamètres et des tailles correspondants aux diamètres et tailles recherchés des pores dans un matériau d'implant, le matériau de cet agent porogène A étant un polymère insoluble dans le au moins un solvant S1 et soluble dans le au moins un solvant S,
le au moins un solvant S dans lequel le polymère biodégradable P est insoluble et le au moins un solvant S dans lequel le matériau de l'agent porogène A est soluble étant identiques,
d) introduction d'au moins 60% en volume, de préférence d'au moins 70% en volume par rapport au volume total du mélange polymère biodégradable P-agent porogène A introduit dans le moule, de microsphères de l'agent porogène A dans un moule ayant la forme et la taille recherchées pour l'implant, ces microsphères formant un empilement compact correspondant à la forme et à la taille des pores à obtenir dans le matériau d'implant,
e) introduction du polymère biodégradable P dans le moule,
f) gélification du mélange obtenu à l'étape e) dans le moule,
g) démoulage du mélange obtenu à l'étape f),
h) élimination de l'agent porogène par lavage avec le au moins un solvant S,
i) réticulation du mélange obtenu à l'étape g),
j) revêtement du mélange obtenu à l'étape i) avec le verre bioactif M ou avec un matériau hybride H formé d'un polymère biodégradable identique ou différent du polymère biodégradable P et du verre bioactif M.

2. Procédé selon la revendication 1, **caractérisé en ce que** l'étape j) est mise en oeuvre par imprégnation du mélange obtenu à l'étape i) avec une suspension, dans un solvant, contenant des particules du verre bioactif M ou du matériau hybride H, et évaporation du solvant.

3. Procédé selon la revendication 1, **caractérisé en ce que** l'étape j) est une étape de revêtement du mélange obtenu à l'étape i) soit avec le verre bioactif M, soit avec le matériau hybride H, et est mise en oeuvre par immersion du mélange obtenu à l'étape i) soit dans un sol contenant les précurseurs alcoxydes du verre bioactif M, pour un revêtement uniquement avec le verre bioactif M, soit dans un sol du matériau hybride, ou dans un sol des précurseurs alcoxydes du verre bioactif M et du polymère biodégradable du matériau hybride H, pour un revêtement avec le matériau hybride H, suivie d'une étape de gélification.

4. Procédé selon l'une quelconque des revendications 1 à 3, **caractérisé en ce que** le polymère biodégradable P est choisi parmi :
- les polymères biodégradables solubles dans un solvant S1 et insolubles dans au moins un solvant S choisis parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables de préférence l'alcool polyvinylique ou le poly(acide lactique),
- les polymères synthétiques biodégradables, de préférence un polyéthylène glycol, ou le poly(caprolactone), ou
- les protéines, de préférence la gélatine ou le collagène,
et **en ce que** le matériau de l'agent porogène A est choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, et l'acrylonitrile butadiène styrène (ABS),
le matériau de l'agent porogène A étant différent du polymère biodégradable P.

5. Procédé selon l'une quelconque des revendications 1 à 4, **caractérisé en ce que** le rapport en masse polymère biodégradable P/ verre bioactif M est compris entre 90/10 et 50/50, bornes incluses, de préférence compris entre 80/20 et 60/40, bornes incluses.

6. Procédé selon l'une quelconque des revendications 1 à 5, **caractérisé en ce que** le verre bioactif M est un verre à base de SiO₂ et de CaO, le polymère biodégradable P est la gélatine, le matériau des microsphères d'agent porogène A est le polyméthacrylate de méthyle, le solvant S est l'acétone et le solvant S1 est l'eau.

7. Procédé selon l'une quelconque des revendications 1 à 6, **caractérisé en ce qu'**il comprend de plus une étape d'introduction d'un agent de couplage, de préférence un composé organoalkoxysilane, plus préférablement du 3-glycidoxypropyltriméthoxysilane (GPTMS), et encore plus préférablement du 3-glycidoxypropyltriéthoxysilane (GPTES) à l'étape e).

8. Procédé selon l'une quelconque des revendications 1 à 7, **caractérisé en ce qu'**il comprend après l'étape d), et avant l'étape e), une étape d'agrandissement des interconnexions (4), par infiltration d'un solvant du matériau de l'agent porogène A, dans l'empilement des microsphères d'agent porogène A et/ou par chauffage de cet empilement.

9. Matériau d'implant, pour le comblement de défauts osseux, la régénération osseuse et l'ingénierie tissulaire de l'os, obtenu par le procédé selon l'une quelconque des revendications 1 à 8,
**caractérisé en ce qu'il comprend :**
• un verre bioactif M à base de SiO₂ et CaO, optionnellement contenant P₂O₅ et/ou optionnellement dopé au strontium, et
• un polymère biodégradable P soluble dans au moins un solvant S1 choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone), et
- les protéines, de préférence la gélatine ou le collagène,
**et en ce que** il consiste en une matrice (1) comprenant au moins le polymère biodégradable P recouvert du verre bioactif M ou d'un matériau hybride H formé du verre bioactif M et d'un polymère biodégradable identique ou différent du polymère biodégradable P, cette matrice (1) ayant au moins 70 % en nombre de pores (2) ayant au moins une interconnexion (4) avec un autre pore et la forme de sphères ou de polyèdres s'inscrivant dans une sphère, le diamètre (3) des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions (4) entre les pores ayant leur plus petite dimension comprise entre 25 µm et 250 µm, bornes incluses.

10. Implant pour le comblement de défauts osseux, la régénération osseuse et l'ingénierie tissulaire de l'os, **caractérisé en ce qu'**il comprend un matériau selon la revendication 9 ou obtenu par le procédé selon l'une quelconque des revendications 1 à 8.

## Patentansprüche

1. Verfahren zur Herstellung eines Implantats für die Überbrückung von Knochendefekten, die Knochenneubildung und die Züchtung von Knochengewebe, **dadurch gekennzeichnet, dass** es folgende Schritte umfasst:
a) Auswahl eines bioaktiven Glases M auf Basis von SiO₂ und CaO, das optional P₂O₅ enthält und/oder optional mit Strontium dotiert ist,
b) Auswahl eines biologisch abbaubaren Polymers P, das in mindestens einem Lösungsmittel S1 löslich und in mindestens einem Lösungsmittel S unlöslich ist,
c) Auswahl von Mikrosphären eines Porenbildners A, die Durchmesser und Größen haben, die den gesuchten Durchmessern und Größen der Poren in einem Implantatmaterial entsprechen, wobei das Material dieses Porenbildners A ein Polymer ist, das in dem mindestens einen Lösungsmittel S1 unlöslich und in dem mindestens einen Lösungsmittel S löslich ist,
wobei das mindestens eine Lösungsmittel S, in dem das biologisch abbaubare Polymer P unlöslich ist, und das mindestens eine Lösungsmittel S, in dem das Material des Porenbildners A löslich ist, identisch sind,
d) Einführung von mindestens 60 Vol.-%, vorzugsweise von mindestens 70 Vol.-% in Bezug auf das Gesamtvolumen des Gemischs biologisch abbaubares Polymer P - Porenbildner A, das in die Form eingeführt wird, von Mikrosphären des Porenbildners A in eine Form, die die gesuchte Gestalt und Größe für das Implantat hat, wobei diese Mikrosphären einen kompakten Stapel bilden, der der Gestalt und der Größe der Poren entspricht, die in dem Implantatmaterial zu erhalten sind,
e) Einführung des biologisch abbaubaren Polymers P in die Form,
f) Gelbildung des in dem Schritt e) erhaltenen Gemischs in der Form,
g) Entformung des in dem Schritt f) erhaltenen Gemischs,
h) Ausscheidung des Porenbildners durch Waschung mit dem mindestens einen Lösungsmittel S,
i) Vernetzung des in dem Schritt g) erhaltenen Gemischs,
j) Beschichtung des in dem Schritt i) erhaltenen Gemischs mit dem bioaktiven Glas M oder mit einem Hybridmaterial H, das aus einem biologisch abbaubaren Polymer, das mit dem biologisch abbaubaren Polymer P identisch ist oder sich von diesem unterscheidet, und dem bioaktiven Glas M gebildet ist.

2. Verfahren nach Anspruch 1, **dadurch gekennzeichnet, dass** der Schritt j) umgesetzt wird durch Imprägnierung des in dem Schritt i) erhaltenen Gemischs mit einer Suspension, in einem Lösungsmittel, die Partikel des bioaktiven Glases M oder des Hybridmaterials H enthält, und Verdampfung des Lösungsmittels.

3. Verfahren nach Anspruch 1, **dadurch gekennzeichnet, dass** der Schritt j) ein Schritt der Beschichtung des in dem Schritt i) erhaltenen Gemischs entweder mit dem bioaktiven Glas M oder mit dem Hybridmaterial H ist und umgesetzt wird durch Eintauchen des in dem Schritt i) erhaltenen Gemischs entweder in ein Sol, das die Alkoxidpräkursoren des bioaktiven Glases M enthält, für eine Beschichtung ausschließlich mit dem bioaktiven Glas M, oder in ein Sol des Hybridmaterials, oder in ein Sol der Alkoxidpräkursoren des bioaktiven Glases M und des biologisch abbaubaren Polymers des Hybridmaterials H, für eine Beschichtung mit dem Hybridmaterial H, gefolgt von einem Schritt der Gelbildung.

4. Verfahren nach einem der Ansprüche 1 bis 3, **dadurch gekennzeichnet, dass** das biologisch abbaubare Polymer P gewählt wird aus :
- biologisch abbaubaren Polymeren, die in einem Lösungsmittel S1 löslich und in mindestens einem Lösungsmittel S unlöslich sind, gewählt aus :
- bioresorbierbaren Polysacchariden, vorzugsweise gewählt aus Dextran, Hyaluronsäure, Agar, Chitosan, Alginsäure, Natrium- oder Kaliumalginat, Galactomannan, Carrageen, Pektin,
- bioresorbierbaren Polyestern, vorzugsweise Polyvinylalkohol oder Poly(milchsäure),
- biologisch abbaubaren synthetischen Polymeren, vorzugsweise Polyethylenglycol oder Poly(caprolacton), oder
- Proteinen, vorzugsweise Gelatine oder Kollagen,
und dadurch, dass das Material des Porenbildners A gewählt wird aus den biologisch abbaubaren Polymeren, die in dem mindestens einen Lösungsmittel S1 unlöslich und in dem mindestens einen Lösungsmittel S löslich sind, vorzugsweise gewählt aus C₁-C₄-Alkylpolymethacrylaten, vorzugsweise Methylpolymethacrylat oder Butylpolymethacrylat, Polyurethan, Polyglycolsäure, unterschiedlichen Formen von Polymilchsäure, Copolymeren von Milchsäure-Co-Glycolsäure, Polycaprolacton, Polypropylenfumarat, Paraffin und Naphtalin, und Acrylnitril-Butadien-Styrol (ABS),
wobei sich das Material des Porenbildners A von dem biologisch abbaubaren Polymer P unterscheidet.

5. Verfahren nach einem der Ansprüche 1 bis 4, **dadurch gekennzeichnet, dass** das Masseverhältnis biologisch abbaubares Polymer P / bioaktives Glas M zwischen 90/10 und 50/50 beträgt, Grenzwerte eingeschlossen, vorzugsweise zwischen 80/20 und 60/40, Grenzwerte eingeschlossen.

6. Verfahren nach einem der Ansprüche 1 bis 5, **dadurch gekennzeichnet, dass** das bioaktive Glas M ein Glas auf Basis von SiO₂ und CaO ist, das biologisch abbaubare Polymer P Gelatine ist, das Material der Mikrosphären des Porenbildners A Methylpolymethacrylat ist, das Lösungsmittel S Aceton ist und das Lösungsmittel S1 Wasser ist.

7. Verfahren nach einem der Ansprüche 1 bis 6, **dadurch gekennzeichnet, dass** es weiter einen Schritt der Einführung eines Haftvermittlers umfasst, vorzugsweise einer organofunktionellen Alkoxysilan-Verbindung, noch bevorzugter 3-Glycidoxypropyltrimethoxysilan (GPTMS) und besonders bevorzugt 3-Glycidoxypropyltriethoxysilan (GPTES), in dem Schritt e).

8. Verfahren nach einem der Absprüche 1 bis 7, **dadurch gekennzeichnet, dass** es nach dem Schritt d) und vor dem Schritt e) einen Schritt der Vergrößerung der Verknüpfungen (4) durch Infiltration eines Lösungsmittel des Materials des Porenbildners A in dem Stapel der Mikrosphären des Porenbildners A und/oder durch Erhitzung dieses Stapels umfasst.

9. Implantatmaterial für die Überbrückung von Knochendefekten, die Knochenneubildung und die Züchtung von Knochengewebe, das erhalten wird durch das Verfahren nach einem der Ansprüche 1 bis 8,
**dadurch gekennzeichnet, dass** es umfasst:
- ein bioaktives Glas M auf Basis von SiO₂ und CaO, das optional P₂O₅ enthält und/oder optional mit Strontium dotiert ist, und
- ein biologisch abbaubares Polymer P, das in mindestens einem Lösungsmittel S1 löslich, gewählt aus:
- bioresorbierbaren Polysacchariden, vorzugsweise gewählt aus Dextran, Hyaluronsäure, Agar, Chitosan, Alginsäure, Natrium- oder Kaliumalginat, Galactomannan, Carrageen, Pektin,
- bioresorbierbaren Polyestern, vorzugsweise Polyvinylalkohol oder Poly(milchsäure),
- biologisch abbaubaren synthetischen Polymeren, vorzugsweise Polyethylenglycol oder Poly(caprolacton), und
- Proteinen, vorzugsweise Gelatine oder Kollagen,
und dadurch, dass es aus einer Matrix (1) besteht, die mindestens das biologisch abbaubare Polymer P umfasst, das mit dem bioaktiven Glas M oder einem Hybridmaterial H überzogen ist, das aus dem bioaktiven Glas M und einem biologisch abbaubaren Polymer, das mit dem biologisch abbaubaren Polymer P identisch ist oder sich von diesem unterscheidet, gebildet ist, wobei diese Matrix (1) anzahlmäßig mindestens 70 % an Poren (2) hat, die mindestens eine Verknüpfung (4) mit einer anderen Pore und die Gestalt von Sphären oder Polyedern haben, die sich in eine Sphäre einfügen, wobei der Durchmesser (3) der Sphären zwischen 100 und 900 µm beträgt, vorzugsweise zwischen 200 und 800 µm, Grenzwerte eingeschlossen, mit einem Abstand zwischen dem Durchmesser der kleinsten oder größten Sphäre von höchstens 70 %, vorzugsweise höchstens 50 %, noch bevorzugter höchstens 30 %, in Bezug auf den arithmetischen mittleren Durchmesser der Gesamtheit der Sphären des Implantats und wobei die Verknüpfungen (4) zwischen den Poren ihre kleinste Dimension bei zwischen 25 µm und 250 µm, Grenzwerte eingeschlossen, haben.

10. Implantat für die Überbrückung von Knochendefekten, die Knochenneubildung und die Züchtung von Knochengewebe, **dadurch gekennzeichnet, dass** es ein Material nach Anspruch 9 umfasst oder durch das Verfahren nach einem der Ansprüche 1 bis 8 erhalten wird.

## Claims

1. Method for manufacturing an implant for filling in bone defects for bone regeneration and bone tissue engineering, **characterised in that** it comprises the following steps:
a) selecting a bioactive glass M based on SiO₂ and CaO, optionally containing P₂O₅ and/or optionally doped with strontium,
b) selecting a biodegradable polymer P which is soluble in at least one solvent S1 and insoluble in at least one solvent S,
c) selecting microspheres of a porogen A having diameters and sizes which correspond to the desired diameters and sizes of the pores in an implant material, the material of said porogen A being a polymer which is insoluble in the at least one solvent S1 and soluble in the at least one solvent S,
wherein the at least one solvent S in which the biodegradable polymer P is insoluble and the at least one solvent S in which the material of the porogen A is soluble are identical,
d) introduction of at least 60% by volume, preferably at least 70% by volume with respect to the total volume of the biodegradable polymer P/porogen A mixture introduced into the mould, of microspheres of the porogen A into a mould having the desired shape and size for the implant, said microspheres forming a compact stack corresponding to the shape and size of the pores to be obtained in the implant material,
e) introducing the biodegradable polymer P into the mould,
f) gelling the mixture obtained in step e) in the mould,
g) removing the mixture obtained in step f) from the mould,
h) eliminating the porogen by washing with the at least one solvent S,
i) crosslinking the mixture obtained in step g),
j) coating the mixture obtained in step i) with the bioactive glass M or with a hybrid material H formed of a biodegradable polymer which is identical to or different from the biodegradable polymer P and bioactive glass M.

2. Method according to claim 1, **characterised in that** step j) is implemented by impregnating the mixture obtained in step i) with a suspension, in a solvent, containing particles of the bioactive glass M or the hybrid material H, and evaporating the solvent.

3. Method according to claim 1, **characterised in that** step j) is a step of coating the mixture obtained in step i) either with the bioactive glass M or with the hybrid material H, and is implemented by immersing the mixture obtained in step i) either in a sol containing the alkoxide precursors of the bioactive glass M for a coating only with the bioactive glass M, or in a sol of the hybrid material, or in a sol of the alkoxide precursors of the bioactive glass M and of the biodegradable polymer of the hybrid material H for a coating with the hybrid material H, followed by a gelling step.

4. Method according to any one of claims 1 to 3, **characterised in that** the biodegradable polymer P is selected from:
- the biodegradable polymers which are soluble in a solvent S1 and insoluble in at least one solvent S selected from:
- bioresorbable polysaccharides, preferably selected from dextran, hyaluronic acid, agar, chitosan, alginic acid, sodium or potassium alginate, galactomannan, carrageenan, pectin,
- bioresorbable polyesters, preferably polyvinyl alcohol or poly(lactic acid),
- biodegradable synthetic polymers, preferably a polyethylene glycol, or poly(caprolactone), or
- proteins, preferably gelatine or collagen,
and the material of the porogen A is selected from biodegradable polymers which are insoluble in the at least one solvent S1 and soluble in the at least one solvent S, preferably selected from C₁ to C₄ polyalkyl methacrylates, preferably polymethyl methacrylate or polybutyl methacrylate, polyurethane, polyglycolic acid, various forms of polylactic acids, lactic-co-glycolic acid copolymers, polycaprolactone, polypropylene fumarate, paraffin and naphthalene, and acrylonitrile butadiene styrene (ABS),
wherein the material of the porogen A is different from the biodegradable polymer P.

5. Method according to any one of claims 1 to 4, **characterised in that** the mass ratio of biodegradable polymer P/bioactive glass M is between 90/10 and 50/50 inclusive, preferably between 80/20 and 60/40 inclusive.

6. Method according to any one of claims 1 to 5, **characterised in that** the bioactive glass M is a glass based on SiO₂ and CaO, the biodegradable polymer P is gelatine, the material of the microspheres of porogen A is polymethyl methacrylate, the solvent S is acetone and the solvent S1 is water.

7. Method according to any one of claims 1 to 6, **characterised in that** it further comprises a step of introducing a coupling agent, preferably an organoalkoxysilane compound, more preferably 3-glycidoxypropyltrimethoxysilane (GPTMS), and more preferably 3-glycidoxypropyltriethoxysilane (GPTES) in step e).

8. Method according to any one of claims 1 to 7, **characterised in that** it comprises, after step d) and before step e), a step of expanding the interconnections (4) by infiltration of a solvent of the material of the porogen A into the stack of microspheres of the porogen A and/or by heating this stack.

9. Implant material, for filling in bone defects, bone regeneration and bone tissue engineering, obtained by the method according to any one of claims 1 to 8,
**characterised in that it comprises:**
• a bioactive glass M based on SiO₂ and CaO, optionally containing P₂O₅ and/or optionally doped with strontium, and
• a biodegradable polymer P which is soluble in at least one solvent S1 selected from:
- bioresorbable polysaccharides, preferably selected from dextran, hyaluronic acid, agar, chitosan, alginic acid, sodium or potassium alginate, galactomannan, carrageenan, pectin,
- bioresorbable polyesters, preferably polyvinyl alcohol or poly(lactic acid),
- biodegradable synthetic polymers, preferably a polyethylene glycol, or poly(caprolactone), and
- proteins, preferably gelatine or collagen,
**and in that** it consists of a matrix (1) comprising at least the biodegradable polymer P covered with bioactive glass M or a hybrid material H formed of bioactive glass M and a biodegradable polymer which is identical to or different from the biodegradable polymer P, said matrix (1) having at least 70% by number of pores (2) having at least one interconnection (4) with another pore and the shape of spheres or polyhedra being embedded in a sphere, the diameter (3) of the spheres being between 100 and 900µm, preferably between 200 and 800µm inclusive, with the difference between the diameter of the smallest sphere and the largest sphere being at most 70%, preferably at most 50%, more preferably at most 30%, with respect to the arithmetic mean diameter of all the spheres of the implant and the interconnections (4) between the pores having their smallest dimension between 25µm and 250µm inclusive.

10. Implant for filling in bone defects, bone regeneration and bone tissue engineering, **characterised in that** it comprises a material according to claim 9 or obtained by the method according to any one of claims 1 to 8.
